(19) Europäisches Patentamt
European Patent Office
Office européen des brevets

(11) **EP 4 537 084 B1**

(12) **EUROPEAN PATENT SPECIFICATION**

(45) Date of publication and mention of the grant of the patent:
**29.04.2026 Bulletin 2026/18**

(21) Application number: **23761248.6**

(22) Date of filing: **13.06.2023**

(51) International Patent Classification (IPC):
**G01N 21/31** *(2006.01)* **G01N 21/85** *(2006.01)*
**G01N 33/493** *(2006.01)*

(52) Cooperative Patent Classification (CPC):
**G01N 21/31; G01N 21/8507; G01N 33/493;**
G01N 2021/3155; G01N 2021/8528

(86) International application number:
**PCT/PL2023/050043**

(87) International publication number:
**WO 2023/244131 (21.12.2023 Gazette 2023/51)**

(54) **A METHOD OF DISCRIMINATING URINARY TRACT INFECTIONS**

EINE METHODE ZUR UNTERSCHEIDUNG VON HARNWEGSINFEKTIONEN

MÉTHODE DE DISCRIMINATION DES INFECTIONS DES VOIES URINAIRES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.06.2022 PL 44149022**

(43) Date of publication of application:
**16.04.2025 Bulletin 2025/16**

(73) Proprietors:
• **Gdanski Uniwersytet Medyczny**
**80-210 Gdansk (PL)**
• **Politechnika Gdanska**
**80-233 Gdansk (PL)**

(72) Inventors:
• **WITYK, Pawel**
**83-330 Lniska (PL)**
• **SOKOLOWSKI, Patryk**
**87-400 Golub-Dobrzyn (PL)**
• **SZCZERSKA, Malgorzata**
**80-180 Gdansk (PL)**
• **MARKUSZEWSKI, Michal**
**80-355 Gdansk (PL)**
• **KRAWCZYK, Beata**
**80-180 Gdansk (PL)**

(74) Representative: **Godlewski, Piotr**
**FGGH IP**
**Kancelaria Patentowa Piotr Godlewski**
**Ul. Bociania 19/8**
**02-807 Warszawa (PL)**

(56) References cited:
**EP-B1- 3 110 963 WO-A1-2018/173073**
**US-A1- 2007 071 648**

**Description**

**Technical Field**

The invention concerns a method of discriminating an urinary tract infections to the extent of differentiating between a urinary tract infection (UTI) and the urinary tract infection able to cause urosepsis. In particular, the invention is directed to a method to analyse an urinary sample to discriminate *E.coli* bacteria able to cause UTI from *E. coli* bacteria which cause urosepsis.

**Background Art**

Sepsis is one of the causes of infections and deaths at hospitals. If sepsis results from a urinary tract infection (UTI), it is referred to as urosepsis. It is estimated that approximately one fourth of the general number of sepsis cases is caused by urinary tract infections, which represents ~12% of these infections. Early administration of proper medication (antibiotic) targeted at the specific causative factor (bacteria) increases the patient's chances to survive. Any delay of one hour or more in launching the therapy may increase the mortality rate from 20% to 33%. The most frequent etiological factors of urosepsis are: Gram-negative bacteria of the Enterobacteriaceae family (*Escherichia, Proteus, Enterobacter, Klebsiella spp.*), as well as non-fermenting bacteria (*Pseudomonas* spp.) and Gram-positive bacteria, less frequently by fungi. Majority of urosepsis cases is caused by the bacteria of the *Escherichia coli* intestinal bacteria.

The method commonly used today to differentiate generalized inflammatory response from sepsis consists in assaying the CRP protein level in combination with assaying the level of procalcitonin (PCT). The *Escherichia coli* bacteria which are the most frequent cause of urosepsis are detected by absorption microscopy using Fourier transform and static analysis.

Known in the prior art are methods of detecting and identifying bacteria.

Disclosed in patent application P.395007 is a method of assaying bacteria colonies cultured on solid media and an optical system to carry out the method.

According to the disclosure, the assay of the cultured bacteria colonies is conducted on solid media, where a collimated beam of light from a coherent light source is filtered using an amplitude filter, polarized, and expanded, whereupon a beam of light is formed using a diaphragm and a lens to the convergent-spherical form and the bacteria colony placed on a transparent medium is lit. The transmitted and deflected beam of light is recorded and the two-dimensional distribution of intensity of the light deflected on the bacteria colony is analyzed.

Disclosed in patent US9970063B2 is a PCR-based test kit and nucleic acid amplification process for identification of sub-species of infectious bacteria, such as uropathogenic E. *coli* causing cystitis, pyelonephritis and sepsis, for the purposes of predicting antibiotic resistance of the bacteria. The clonal E. coli strains are differentiated based on SNP (single nucleotide polymorphism) identification.

Disclosed in patent EP 3 110 963 B1 is a method of analysing a sample including a microorganism of interest, such as a bacterium responsible for causing a urinary tract infection ("UTI"). UV-Vis spectroscopy and other spectroscopic techniques (such as FT-IR and Raman) is used to obtain a fast and accurate diagnosis of the susceptibility/resistance of bacteria responsible for causing UTIs. to antibiotics. using a simple and inexpensive screening tool.

In order to diagnose urinary tract infections and implement an appropriate treatment as soon as possible, it is necessary to provide a swift and cost-effective method of diagnosing infections of the type, in particular a method which will enable differentiation between uropathogenic E. *coli* strains.

It has been found in the research works conducted by the Inventors that it is possible to differentiate uropathogenic E. *coli* strains using optical methods, where analysis is conducted on a liquid medium.

**Summary of Invention**

The invention, defined by appended independent claim 1, concerns the provision of a method to analyse an urinary sample to discriminate *E. coli* bacteria able to cause urinary tract infection (UTI) from

*E. coli* bacteria which cause urosepsis using an optical system which employs measurements of absorbance of waves of the visible spectrum. The specific changes in the absorbance spectrum which occurred between the patient groups and control groups made it possible to develop an algorithm which differentiates between these two populations based on the recorded spectrum.

In greater detail, the invention, defined by appended independent claim 1, concerns a method to analyse an urinary sample 3 to discriminate *E. coli* bacteria able to cause urinary tract infection (UTI) from *E. coli* bacteria which cause urosepsis using a system consisting of a light source 1 which emits radiation having a central wavelength within the range of 200 - 780nm, a cuvette 2 into which an urine sample 3 to be tested is inserted or a container 2 with the urine sample 3 to be tested into which a measuring probe 6 is dipped, a detector 4, where the detector 4 is placed at a distance from the light source 1, and an analyzer 5 programmed to classify the urine sample to UTI/urosepsis based on the reading of the differentiating factor R, the method according to the invention, wherein the method progresses in the following stages:

a) reference urine samples from patient groups of different age groups and sexes, with infections confirmed on genetically verified clinical strains representing confirmed UTI caused by *E.coli* and patients with confirmed Urosepsis caused by *E.coli* are provided;

b) light absorbance measurements at the wavelength of 600 nm are performed for the reference urine samples and a differentiating factor R characteristic for UTI and Urosepsis infections is determined for each patient group so as to achieve 95% likelihood that it would indicate the correct UTI/Urosepsis group;

c) from a patient's sterile urine container a specific volume of the patient's urine sample 3 is collected into the cuvette 2 or the measuring probe 6 is dipped in the urine, whereupon urine absorbance is measured in the radiation of the central wavelength of 200-780 nm emitted by the light source 1, wherein before measuring the absorbance, the urine sample is centrifuged to separate the supernatant from the sediment at 4 000 x g for 20 min at the temperature of 25°C, and wherein the preliminary selection of the urine samples is conducted using measurement of sample absorbance at the central wavelength of 600 +/- 20 nm, thus determining the normalizing parameter N, where referred for further analysis is the urine sample for which the parameter N is higher than 0.2;

d) the light absorbance is measured using the detector 4,

e) stages c) and d) are carried out for the entire range of 200-780 nm, thus obtaining the dependence of absorbance A on wavelength $\lambda$ and the absorbance measurement results are transmitted to the analyzer 5, wherein the analyzer 5 determines the differentiating factor R by using the absorbance measurement results from the entire range of the 200-780 nm and calculating the surface area P from the following formula: $P = \int_{600}^{750} A(\lambda)\, d\lambda$ then calculating the differentiating factor R from the formula $R \begin{cases} P/N < 0, -Log(|P/N|) \\ P/N > 0, Log(|P/N|) \end{cases}$ and, based on the differentiating factor R, the sample is classified to UTI/urosepsis by the analyzer 5.

Preferably, in the method according to the invention the differentiating factor R above 2 indicates that the sample was collected from a patient of the UTI group, factor R below -2 indicates that the sample was collected from a patient of the Urosepsis group, and factor R of <-2, 2> indicates an uncertain result.

Preferably, in the method according to the invention the differentiating factor R is determined for a reference liquid culture on genetically verified clinical strains or for urine samples from patients with confirmed infections, namely with confirmed UTI caused by *E. coli* and with Urosepsis caused by *E. coli,* respectively.

Compared to the solution disclosed in application P.395007, which presented a method of assaying bacteria colonies cultured on solid media and an optical system for carrying out the method and enabling differentiation between the assayed groups using measurements within the wavelength of 670 nm, the solution according to the invention, defined by claim 1, does not require culturing a colony on a solid base, and in addition used in the measuring system according to the present disclosure are measurements of sample absorbance instead of reflected light measurements, as is the case of the cited disclosure. Moreover, in the solution according to the present disclosure additional absorbance measurements are taken before and after the sample is centrifuged, which is vital for the correct interpretation of the results and differentiation between the groups. In addition, in the method according to the invention collected is a urine sample instead of blood sample, as in P.395007, hence the method according to the invention is less invasive and enables taking the measurements without the need for the presence of diagnostic laboratory personnel, the latter required to take a blood sample. Moreover, in accordance with the method according to the invention, defined by claim 1, absorbance measurements may be taken using both a regular spectrometer, and a solution based on measurement probe used to measure absorbance in in liquid samples, hence the measuring system in the method according to the invention is simplified, and sample analysis is less time consuming and less costly than that in the said disclosure.

According to the present disclosure two methods of preparing samples for testing were proposed, namely directly from patient's urine sample and by growing a culture on liquid medium inoculated with patient's urine. The method according to the invention, defined by claim 1, can be used to perform a swift and reliable discrimination of urinary tract infection from an infection able to cause urosepsis, thanks to which it is possible to take up proper treatment early.

The advantages of the proposed solution consist primarily in the swiftness of taking the measurement, mobility of the measurement-taking method (no medical personnel required to collect the sample), and the low cost of the test. The solution according to the invention can be used in medical laboratories and medical and veterinary clinics. Contrary to the solutions known in the prior art, where the differentiating method consists in testing the radiation dispersed on a solid medium, the solution according to the invention is based on measuring changes in the intensity of light in the visible spectrum, once it has passed through the liquid medium (urine, liquid bacteria culture, or any other liquid) without the need to grow a culture on a solid medium, which requires at least 24h of sample incubation and qualified personnel able to identify bacteria based on the colony phenotype.

As mentioned above, the solution according to the invention eliminates the need to collect a sample, i.e. patient's blood, by an invasive method. Currently, in order to classify a patient to the UTI or Urosepsis group, diagnostic laboratories grow cultures from blood and urine on a solid medium and differentiation is only possible afterwards. In accordance with the invention it is possible to classify a patient in a respective group solely based on the observations of changes in urine sample absorbance.

The solution according to the invention can be used in medical laboratories and medical and veterinary clinics.

Brief Description of Drawings

Embodiments of the solution according to the invention is shown on drawing figures, where

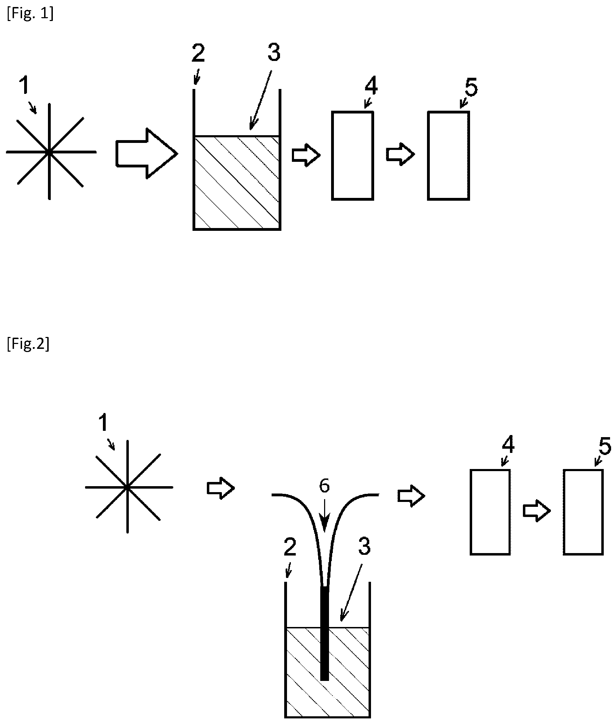

[Fig.1] shows a diagram of the arrangement of the measuring system to carry out the method according to an embodiment of the invention in the variant with a cuvette. The references used in the figure have the following meanings: 1 - light source, 2 - plastic cuvette, 3 - sample, 4 - optical radiation detector and 5 - data analyser.

[Fig.2] shows a diagram of the arrangement of the measuring system to carry out the method according to an embodiment of the invention in the variant with a measuring probe. The references used in the figure have the following meanings: 1 - light source, 2 - container, 3 - sample, 4 - optical radiation detector, 5 - data analyser, 6- measuring probe.

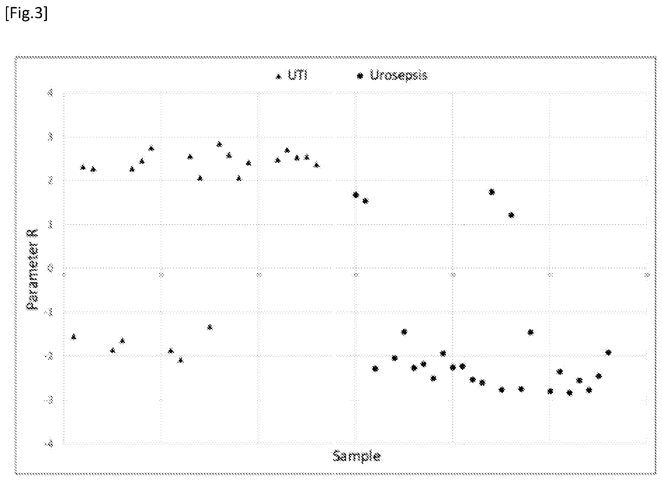

[Fig.3] shows the distribution of factor R calculated for cultures of clinical E. *coli* strains, as detailed in the description of embodiments of the invention.

## Description of Embodiments

The present invention is presented in more detail in embodiments which do not limit its scope. The scope of the present invention is solely limited and defined by the appended claims.

## Examples

In order to differentiate E. *coli* bacteria able to cause UTI from *E. coli* bacteria which cause urosepsis it is necessary to perform: (i) reference measurements which enable determination of the R parameter depending on the culture medium used or the regional changes in absorbance resulting from different diets of the patients if the measurements are conducted on patients' urine samples; (ii) - proper measurements which enable classifying patients in the UTI or Urosepsis group.

Sample preparation

### A. Patient's urine

A sample of the patient's urine was collected in a sterile container. Then, 2 mL of the urine was collected and transferred to a measurement cuvette.

The sample was placed in a measuring device where the source of radiation was a diode emitting a wave of the central wavelength of at least 600 +/- 20 nm.

Then, the measurement was taken and the absorbance read for the wavelength of 600 nm, thus obtaining the normalising parameter N. Parameter N > 0.2 indicates that the biological material can be used for further analyses.

Subsequently, 3 mL of urine was collected again from the sterile container with the patient's urine and centrifuged to separate the supernatant from the sediment (4 000 x g, 20 min, 25°C).

The supernatant was the sample subject to absorbance measurements.

### B. Liquid culture

With the view of performing inoculation, a solution of the liquid medium was prepared as follows.

Added to 1 litre of ultrapure water were: 6g/L of disodium hydrogen phosphate, 3g/L of potassium dihydrogen phosphate, 0,5g/L of sodium chloride, and 1g/L of ammonium chloride, and mixed until the components dissolved. The final pH of the solution was identified as 7.4 at 25°C. Then, the solution was autoclaved at 121°C for 15 min, following which added were: 2 mL of 1M magnesium sulphate solution, and 20 mL of 20% glycerol solution, where both solutions had been first subject to sterilisation by filtration through a filter with pores of the diameter of 0.2 $\mu$m.

Next, a sample of the patient's urine was collected in a sterile container and inoculated. To that aim, 2 mL of the sampled urine was collected and added to 8mL of sterile liquid medium. Then, culturing was performed for 24h at 37 °C shaking it at the speed of 70 rpm.

Once the culturing was completed, 2 mL of the culture was collected, the sample placed in the measuring device where the source of radiation was a diode emitting the wave of the central wavelength of at least 600 +/- 20 nm.

Then, the absorbance read for the wavelength of 600 nm, thus obtaining the normalising parameter N, where parameter N of > 0.2 indicates that the biological material can be used for further analyses.

Next, a sample of 3 mL of the culture was collected again and centrifuged to separate the supernatant from the sediment (4 000 x g, 20 min, 25 °C). The supernatant was the sample subject to the absorbance measurements.

Measurement

The measurement system incorporates such devices as an optical radiation detector (e.g. optical power meter), and a source of radiation. The radiation source and the detector are arranged as appropriate so that having passed through the tested sample the light falls on the detector (the measurement is conducted by analysing the beam of unreflected light - opposite to invention P.395007 where analysed is the dispersed radiation), then the data from the detector are directed to the analyser which processes the obtained data. The diagram of the measuring system is presented on Fig.1.

The measurement was performed so that radiation of the central wavelength of 200-780nm was emitted from the source of light, i.e. a diode, the beam of light was directed onto a plastic cuvette 2 containing the tested sample 3, and having passed through cuvette 2 the beam of light fell on the detector 4 placed at a specific distance from the source of light 1, the absorbance of the sample for the given wavelength is then read in the detector 4, the reading data are recorded in the analyser which, based on the differentiating factor R obtained for the reference samples, classifies the samples as UTI/Urosepsis.

The absorbance was measured for the whole range of wavelength 200-780 nm obtaining the dependence of absorbance A on wavelength $\lambda$ and the surface area P was calculated from the following formula: $P = \int_{600}^{750} A(\lambda)\, d\lambda$

Finally, the differentiating factor R was calculated from the following formula:

$$R = \begin{cases} P/N < 0, -Log(|P/N|) \\ P/N > 0, Log(|P/N|) \end{cases}$$

Reference measurements

Reference measurements were performed in order to determine the differentiating factor R characteristic for UTI and Urosepsis infections. The measurement of sample absorbance was taken at the wavelength of 600 nm.

The tested urine samples came from 50 patients of different age groups and sexes, with infections confirmed on genetically verified clinical strains representing, as appropriate, confirmed UTI caused by *E. coli* and patients with confirmed Urosepsis caused by *E. coli.*

Liquid cultures were grown for all collected samples (according to the procedure described in pt. B) using the collected urine, and absorbance was measured for each of the obtained samples as described above. The results are presented on Fig.3.

Based on an analysis of the reference results the ranges of the R factor were determined for each patient group so as to achieve 95% likelihood that it would indicate the correct UTI/Urosepsis group.

It was established that whenever R > 2 one should assume that the patient represents the UTI patient group, whereas for R <-2 one should assume that the patient falls in the Urosepsis patient group. The R results in the range of <-2, 2> were assumed less diagnostic, hence defined as uncertain results.

Proper measurement

Once the diagnostic range of parameter R had been determined for the reference samples, proper measurements were performed for the urine samples from patients suffering from urinary tract infection of unknown origin to discriminate between UTI/Urosepsis.

The urine samples were prepared according to the procedure described above (pt. A or B)

The results of the measurements taken on the urine sampled from the patients are presented in Table 1.

Table 1

| Patient | N | Sample preparation procedure | R | UTI | Urosepsis | Uncertain |
|---|---|---|---|---|---|---|
| 1 | >0.2 | A | 1.65 | | | + |
| 2 | >0.2 | A | 2.70 | + | | |
| 3 | >0.2 | A | - 0.21 | | | + |
| 4 | >0.2 | A | - 2.40 | | + | |
| 5 | >0.2 | B | - 1.12 | | | + |
| 6 | >0.2 | B | - 2.34 | | + | |
| 7 | >0.2 | B | 2.21 | + | | |
| 8 | >0.2 | B | 2.09 | + | | |
| 9 | >0.2 | B | 0.34 | | | + |
| 10 | >0.2 | B | 2.11 | + | | |
| 11 | >0.2 | A | - 2.18 | | + | |

In order to validate the method, absorbance measurements were taken for ultrapure water, urine from a healthy patient, and culture medium M9, with the obtained results presented in Table 2.

Table 2.

| Sample | N | R | UTI | Urosepsis | Uncertain |
|---|---|---|---|---|---|
| H2O | <0.2 | None | | | + |
| Healthy | <0.2 | None | | | + |
| M9 | <0.2 | none | | | + |

**Claims**

1.   A method to analyse an urinary sample (3) to discriminate E. coli bacteria able to cause urinary tract infection (UTI) from *E. coli* bacteria which cause urosepsis using a system consisting of a light source (1) which emits radiation having a central wavelength within the range of 200-780nm, a cuvette (2) into which an urine sample (3) to be tested is inserted or a container (2) with an urine sample (3) to be tested into which a measuring probe (6) is dipped, a detector (4), wherein the detector (4) is placed at a distance from the light source (1), and an analyzer (5) programmed to classify the urine sample (3), wherein the method progresses in the following stages:

a) reference urine samples from patient groups of different age groups and sexes, with infections confirmed on genetically verified clinical strains representing confirmed UTI caused by *E.coli* and patients with confirmed Urosepsis caused by *E.coli* are provided;

b) light absorbance measurements at the wavelength of 600 nm are performed for the reference urine samples and a differentiating factor R characteristic for UTI and Urosepsis infections is determined for each patient group so as to achieve 95% likelihood that it would indicate the correct UTI/Urosepsis group;

c) from a patient's sterile urine container, a specific volume of the patient's urine is collected into the cuvette (2) as an urine sample (3) to be tested; alternatively, the measuring probe (6) is dipped in a specific volume of the patient's urine in the container (2) as sample (3) to be tested, whereupon urine absorbance is measured using the radiation having the central wavelength within the range of 200-780 nm emitted by the light source (1), wherein before measuring the absorbance, the urine sample (3) is centrifuged to separate the supernatant from the sediment at 4 000 x g for 20 min at the temperature of 25°C, and wherein the preliminary selection of the patient's urine is conducted by measuring the absorbance of the urine sample at the central wavelength of 600 +/- 20 nm, thus determining a normalising parameter N, where referred for further analysis is the urine sample (3) for which parameter N is greater than 0.2;

d) the light absorbance is measured using the detector (4),

e) stages c) and d) are carried out for the entire range of 200-780 nm, thus obtaining the dependence of absorbance A on wavelength $\lambda$, and the absorbance measurement results are transmitted to the analyzer (5), wherein the analyzer (5) determines the differentiating factor R by using the absorbance measurement results from the entire range of the 200-780 nm and calculating the surface area P from the formula $\int_{600}^{750} A(\lambda)\, d\lambda$,

then calculating the differentiating factor R from the

$$\text{formula } R \begin{cases} P/N < 0, -Log(|P/N|) \\ P/N > 0, Log(|P/N|) \end{cases},$$

and based on the determined differentiating factor R, classifying the sample to UTI/Urosepsis.

2. A method according to Claim 1 wherein a differentiating factor R above 2 indicates that the sample was collected from a patient of the UTI group, and a differentiating factor R below -2 indicates that the sample was collected from a patient of the Urosepsis group, and factor R of <-2, 2> indicates an uncertain result.

3. A method according to Claim 2 wherein the differentiating factor R is determined for a reference liquid culture on genetically verified clinical strains or for urine samples from patients with confirmed infections, namely, with confirmed UTI caused by *E. coli* and patients with confirmed Urosepsis caused by *E. coli,* respectively.

**Patentansprüche**

1. Verfahren zur Analyse einer Urinprobe (3) zur Unterscheidung von E. coli-Bakterien, die Harnwegsinfektion verursachen können, von E. coli-Bakterien, die eine Urosepsis verursachen, unter Verwendung eines Systems, bestehend aus einer Lichtquelle (1), die Strahlung mit einer zentralen Wellenlänge im Bereich von 200-780 nm emittiert, einer Küvette (2), in die eine zu untersuchende Urinprobe (3) eingebracht wird, oder einem Küvette (2) mit einer zu untersuchenden Urinprobe (3), in den eine Messsonde (6) eingetaucht wird, und einem Detektor (4), wobei der Detektor (4) in einem Abstand von der Lichtquelle (1) angeordnet ist, und einem Analysator (5), der so programmiert ist, dass er die Urinprobe (3) klassifiziert, wobei das Verfahren in den folgenden Schritten abläuft:

a) Es werden Referenzurinproben von Patientengruppen unterschiedlichen Alters und Geschlechts bereitgestellt, bei denen Infektionen durch genetisch verifizierte klinische Stämme bestätigt wurden, die für eine durch E. *coli* verursachte bestätigte Harnwegsinfektion stehen, sowie von Patienten mit einer durch E. *coli* verursachten bestätigten Urosepsis;

b) Für die Referenzurinproben werden Lichtabsorptionsmessungen bei einer Wellenlänge von 600 nm durchgeführt, und für jede Patientengruppe wird ein für Harnwegsinfektionen und Urosepsis-Infektionen charakteristisches Unterscheidungsmerkmal R ermittelt, sodass eine Wahrscheinlichkeit von 95 % erreicht wird, dass die richtige Harnwegsinfektions-/Urosepsis-Gruppe identifiziert wird;

c) Aus dem sterilen Urinbehälter eines Patienten wird eine bestimmte Menge des Urins des Patienten als zu untersuchende Urinprobe (3) in die Küvette (2) aufgefangen; alternativ wird die Messsonde (6) in ein bestimmtes Volumen des Urins des Patienten in die Küvette (2) als zu untersuchende Urinprobe (3) getaucht, woraufhin die Urinabsorption unter Verwendung der von der Lichtquelle (1) emittierten Strahlung mit einer zentralen Wellenlänge im Bereich von 200-780 nm gemessen wird, wobei vor der Messung der Absorption die Urinprobe (3) zentrifugiert wird, um den Überstand vom Sediment bei 4 000 x g für 20 min bei einer Temperatur von 25 °C zu trennen, und wobei die Vorauswahl des Urins des Patienten durch Messung der Absorption der Urinprobe bei der zentralen Wellenlänge von 600 $\pm$ 20 nm erfolgt, wodurch ein Normalisierungsparameter N bestimmt wird, wobei zur weiteren Analyse die Urinprobe (3) weitergeleitet wird, für die der Parameter N größer als 0,2 ist;

d) die Lichtabsorption wird mit dem Detektor (4) gemessen,

e) Die Schritte a) und d) werden für den gesamten Wellenlängenbereich von 200 bis 780 nm durchgeführt, wodurch die Abhängigkeit der Absorption A von der Wellenlänge $\lambda$ ermittelt wird, und die Absorptionsmesswerte an den Analysator (5) übertragen werden, wobei der Analysator (5) den Unterscheidungsmerkmal R bestimmt, indem er die Absorptionsmesswerte aus dem gesamten Bereich von 200-780 nm verwendet und die Oberfläche P aus der Formel $\int_{600}^{750} A(\lambda)\,d\lambda$ , anschließend den Unterscheidungsmerkmal R anhand der Formel

$$R \begin{cases} P/N < 0, -Log(|P/N|) \\ P/N > 0, Log(|P/N|) \end{cases}$$

, berechnet und auf der Grundlage des Unterscheidungsmerkmals R die Probe als Harnwegsinfektion/Urosepsis klassifiziert.

2. Verfahren nach Patentanspruch 1, wobei ein Unterscheidungsmerkmal R über 2 anzeigt, dass die Probe von einem Patienten der Gruppe für Harnwegsinfektion entnommen wurde, und ein Unterscheidungsmerkmal R unter -2 anzeigt, dass die Probe von einem Patienten der Urosepsis-Gruppe entnommen wurde, und ein Merkmal R von <-2, 2> ein ungewisses Ergebnis anzeigt.

3. Verfahren nach Patentanspruch 2, wobei das Unterscheidungsmerkmal R für eine Referenz-Flüssigkultur auf genetisch verifizierten klinischen Stämmen oder für Urinproben von Patienten mit bestätigten Infektionen bestimmt wird, und zwar von Patienten mit bestätigter, durch E. *coli* verursachter Harnwegsinfektion bzw. von Patienten mit bestätigter, durch E. *coli* verursachter Urosepsis.

**Revendications**

1. Une méthode d'analyse d'un échantillon urinaire (3) permettant de distinguer les bactéries E. *coli* susceptibles de provoquer une infection urinaire (IU) des bactéries E. *coli* responsables d'une urosepsie, à l'aide d'un système composé d'une source lumineuse (1) émettant un rayonnement dont la longueur d'onde centrale est comprise dans la plage de 200 à 780 nm, d'une cuvette (2) dans laquelle est inséré un échantillon d'urine (3) à analyser, ou d'un récipient (2) contenant un échantillon d'urine (3) à analyser dans lequel est plongée une sonde de mesure (6), d'un détecteur (4) placé à distance de la source lumineuse (1), et d'un analyseur (5) programmé pour classifier l'échantillon d'urine (3), la méthode se déroulant selon les étapes suivantes :

   a) des échantillons d'urine de référence provenant de groupes de patients de différents groupes d'âge et sexes, avec des infections confirmées sur des souches cliniques génétiquement vérifiées représentant des infections urinaires confirmées causées par E. *coli* et des patients présentant une urosepsie confirmée causée par E. *coli* sont fournis ;
   b) des mesures d'absorbance lumineuse à la longueur d'onde de 600 nm sont effectuées sur les échantillons d'urine de référence, et un facteur de différenciation R caractéristique des infections IU et Urosepsis est déterminé pour chaque groupe de patients, de manière à obtenir une probabilité de 95 % d'indiquer correctement le groupe IU/Urosepsis ;
   c) à partir du récipient d'urine stérile d'un patient, un volume spécifique d'urine du patient est prélevé dans la cuvette (2) en tant qu'échantillon d'urine (3) à analyser ; alternativement, la sonde de mesure (6) est plongée dans un volume spécifique d'urine du patient dans le récipient (2) en tant qu'échantillon (3) à analyser, après quoi l'absorbance de l'urine est mesurée à l'aide du rayonnement de longueur d'onde centrale comprise dans la plage de 200 à 780 nm émis par la source lumineuse (1), étant précisé qu'avant la mesure de l'absorbance, l'échantillon d'urine (3) est centrifugé afin de séparer le surnageant du sédiment à 4 000 x g pendant 20 min à une température de 25 °C, et que la présélection de l'urine du patient est effectuée en mesurant l'absorbance de l'échantillon d'urine à la longueur d'onde centrale de 600 +/- 20 nm, déterminant ainsi un paramètre de normalisation N, seul l'échantillon d'urine (3) pour lequel le paramètre N est supérieur à 0,2 étant transmis pour analyse ultérieure;
   d) l'absorbance lumineuse est mesurée à l'aide du détecteur (4),
   e) les étapes a) et d) sont réalisées sur l'ensemble de la plage de 200 à 780 nm, obtenant ainsi la dépendance de l'absorbance A en fonction de la longueur d'onde λ, et les résultats des mesures d'absorbance sont transmis à l'analyseur (5), lequel détermine le facteur de différenciation R en utilisant les résultats des mesures d'absorbance sur l'ensemble de la plage de 200 à 780 nm, en calculant la surface P à partir de la formule

   $\int_{600}^{750} A(\lambda)\,d\lambda$ , puis en calculant le facteur de différenciation R à partir de la formule

   $$R \begin{cases} P/N < 0, -Log(|P/N|) \\ P/N > 0, Log(|P/N|) \end{cases}$$

   , et en classifiant l'échantillon dans le groupe IU/Urosepsis sur la base du facteur de différenciation R.

**2.** Une méthode selon la Revendication 1, dans laquelle un facteur de différenciation R supérieur à 2 indique que l'échantillon a été prélevé chez un patient du groupe IU, un facteur de différenciation R inférieur à -2 indique que l'échantillon a été prélevé chez un patient du groupe Urosepsis, et un facteur R compris dans l'intervalle <-2, 2> indique un résultat incertain.

**3.** Une méthode selon la Revendication 2, dans laquelle le facteur de différenciation R est déterminé pour une culture liquide de référence sur des souches cliniques génétiquement vérifiées ou pour des échantillons d'urine de patients présentant des infections confirmées, à savoir respectivement des patients avec une IU confirmée causée par E. *coli* et des patients avec une urosepsie confirmée causée par *E. coli.*

[Fig. 1]

[Fig.2]

[Fig.3]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 395007 A
- US 9970063 B2

- EP 3110963 B1